# EUROPÄISCHE PATENTSCHRIFT

(11) **EP 3 929 177 B1**
(45) Veröffentlichungstag und Bekanntmachung des Hinweises auf die Patenterteilung: **29.11.2023**
(21) Anmeldenummer: 20182431.5
(22) Anmeldetag: 26.06.2020
(51) Int. Cl.: C07C 263/04, C07C 265/14, C07C 273/18, C07C 275/16, C07C 269/00, C07C 271/22

(54) **VERFAHREN ZUR HERSTELLUNG VON DIISOCYANATEN AUF BASIS VON LYSIN**
METHOD FOR THE PREPARATION OF DIISOCYANATES BASED ON LYSINE
PROCÉDÉ DE FABRICATION DE DIISOCYANATES À BASE DE LYSINE

(43) Veröffentlichungstag der Anmeldung: 29.12.2021
(73) Patentinhaber: Evonik Operations GmbH, 45128 Essen (DE)
(72) Erfinder: SPYROU, Emmanouil, 46514 Schermbeck (DE); LOESCH, Holger, 44627 Herne (DE); KREISCHER, Susanne, 45701 Herten (DE); DIESVELD, Andrea, 48712 Gescher (DE); THESING, Andrea, 48683 Ahaus (DE); NITZ, Jörg-Joachim, 45257 Essen (DE)
(74) Vertreter: Evonik Patent Association

(56) Entgegenhaltungen:
- EP-A1- 3 626 705
- V. F. GONKO ET AL: "Synthesis and investigation of alpha-nitrosoureidocarbonic acids with potential antitumor activity", PHARMACEUTICAL CHEMISTRY JOURNAL, Bd. 12, Nr. 5, 1. Mai 1978 (1978-05-01), Seiten 601-606, XP55754443, US ISSN: 0091-150X, DOI: 10.1007/BF00777980

## Beschreibung

Die vorliegende Erfindung betrifft ein Verfahren zur Herstellung eines Diisocyanats und das damit hergestellte Diisocyanat.

### HINTERGRUND DER ERFINDUNG

Diisocyanate auf Basis von Lysin (auch als "Lysindiisocyanatester" im Stand der Technik bezeichnet) sind bekannte Verbindungen und werden in erster Linie für medizinische Anwendungen eingesetzt. Für diese Verbindungen sind im Stand der Technik verschiedene Herstellungsverfahren offenbart.

EP 3 527 593 A1 lehrt ein Verfahren zu ihrer Herstellung unter Verwendung von Phosgen. Aufgrund der ausgeprägten Toxizität dieser Verbindung sind aufwändige und kostenintensive Sicherheitsvorkehrungen während der Lagerung und der Produktion erforderlich. Neben den Gesundheits- und Umweltaspekten sind solche aufwändigen und kostenintensiven Sicherheitsvorkehrungen aber ungewünscht, da sie letztlich die Wirtschaftlichkeit des Verfahrens beeinträchtigen. Auch gängige Alternativen zu Phosgen wie beispielsweise Triphosgen weisen eine ähnliche Toxizität auf. Daher gilt für diese Verbindungen und Verfahren, die sie einsetzen, das oben genannte analog.

EP 3 626 705 A1 offenbart ein Verfahren zur Herstellung von Lysindiisocyanatestern aus Lysin. Weitere Verfahren des Standes der Technik weisen darüber hinaus auch den Nachteil auf, dass die Diisocyanate ungewünschte Farben aufweisen. Um diese Farben zu entfernen sind aufwändige Aufreinigungsverfahren erforderlich, so dass die Wirtschaftlichkeit solcher Verfahren nicht gegeben ist.

### AUFGABE DER ERFINDUNG

Aus dem Stand der Technik bekannte Verfahren weisen unter anderem den Nachteil auf, dass sie sehr giftige und ökologisch bedenkliche Verbindungen einsetzen. Dies ist aber nicht mit den Anforderungen einer nachhaltigeren Wirtschaft vereinbar.

Deshalb besteht die Notwendigkeit, die Nachteile des Stands der Technik zu überwinden und ein verbessertes Verfahren zur Herstellung des Diisocyanats der Formel (A) bereitzustellen. Diese Verbesserungen zielen auf eine verbesserte Umweltverträglichkeit und geringere Gesundheitsrisiken des Verfahrens ab. Auch soll das Verfahren idealerweise vereinfacht werden, insbesondere im Hinblick auf die erforderlichen Sicherheitserfordernisse zu seiner Durchführung. Letztlich ist auch gewünscht die Wirtschaftlichkeit des Verfahrens zu verbessern, zum Beispiel durch die Verwendbarkeit einfacher Anlagen oder durch die Vermeidung aufwändiger Aufreinigungsverfahren.

### ZUSAMMENFASSUNG DER ERFINDUNG

Die der vorliegenden Erfindung zugrunde liegenden Aufgaben werden gelöst durch das erfindungsgemäße Verfahren zur Herstellung eines Diisocyanats der Formel (A) 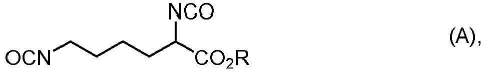 wobei R aus der Gruppe bestehend aus Alkyl, Aryl und Molekülfragmenten, die sowohl wenigstens eine Alkylgruppe und wenigstens eine Arylgruppe umfassen, ausgewählt werden, umfassend die Verfahrensschritte in der angegebenen Reihenfolge:
1) Bereitstellen eines Zwischenprodukts der Formel (B) 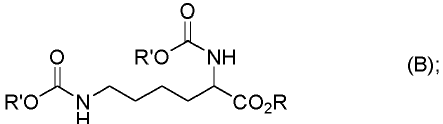 wobei R und jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird; und
2) Thermolytische Spaltung des Zwischenprodukts der Formel (B), so dass das Diisocyanat der Formel (A) erhalten wird,
dadurch gekennzeichnet, dass dem Verfahrensschritt 1 eine der drei weiter unten definierten Varianten 1, 2 oder 3 vorangestellt wird, um das Zwischenprodukt der Formel B bereitzustellen.

### BESCHREIBUNG DER ERFINDUNG

Prozentangaben in der Beschreibung und in den Ansprüchen sind Gewichtsprozent (abgekürzt als Gew.-%), sofern nichts anderes bestimmt ist. Ausbeuten werden als Prozentsatz der theoretischen Ausbeute angegeben. Die in der Folge beschriebenen unterschiedlichen Ausführungsformen können miteinander kombiniert werden, sofern dies technisch möglich und nichts Gegenteiliges bestimmt wird. Die Begriffe "Umsetzung" und "Reaktion" werden wie im Stand der Technik üblich synonym verwendet.

Der Begriff "Alkyl" im Sinne der vorliegenden Erfindung umfasst verzweigte und unverzweigte Alkylgruppen. C1-CX-Alkyl in der Beschreibung und in den Ansprüchen bezieht sich auf Alkylgruppen umfassend 1 bis X Kohlenstoffatome (X ist eine natürliche Zahl). C1-C8-Alkyl schließt beispielsweise Methyl, Ethyl, n-Propyl, iso-Propyl, n-Butyl, iso-Butyl, sec-Butyl, tert-Butyl, n-Pentyl, iso-Pentyl, sec-Pentyl, tert-Pentyl, neo-Pentyl, Hexyl, Heptyl und Octyl mit ein.

Der Begriff "Aryl" im Sinne der vorliegenden Erfindung umfasst ringförmige, aromatische Molekülfragmente (oder Gruppen), zum Beispiel Phenyl oder Naphthyl.

Beispiele für Molekülfragmente, die sowohl wenigstens eine Alkylgruppe und wenigstens eine Arylgruppe umfassen, sind Benzyl und Toloyl.

Optional sind Alkyl und Aryl funktionalisiert. Dabei wird formal ein Wasserstoffatom der genannten Gruppe durch eine funktionelle Gruppe ausgetauscht, vorzugsweise durch Hydroxy- (-OH) und/oder Amino (-NH₂)-Gruppen.

Insofern für eine in den Ansprüchen oder in der Beschreibung genannte Verbindung mehr als Rest ausgewählt werden muss - gleich ob aus einer oder mehreren Listen -, so werden diese Reste unabhängig voneinander ausgewählt. Sie können, sofern in den Listen vorgesehen, somit gleich oder verschieden sein.

Insofern in den Ansprüchen oder in der Beschreibung das Zahlwort "eins" (zum Beispiel "ein Diisocyanat" oder "ein Alkohol") verwendet wird, so ist darunter durchgängig zu verstehen, dass "wenigstens eins" gemeint ist (zum Beispiel "wenigstens ein Diisocyanat" oder "wenigstens ein Alkohol"), also eins oder mehr als eins. Zur Verbesserung der Lesbarkeit wurde auf diese Formulierung verzichtet.

Das erfindungsgemäße Verfahren eignet sich zur Herstellung eines Diisocyanats der Formel (A) 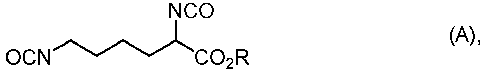 wobei R aus der Gruppe bestehend aus Alkyl, Aryl und Molekülfragmenten, die sowohl wenigstens eine Alkylgruppe und wenigstens eine Arylgruppe umfassen, ausgewählt wird.

R wird bevorzugt aus der Gruppe bestehend C1-C8-Alkyl ausgewählt, mehr bevorzugt aus Methyl, Ethyl, iso-Propyl, n-Propyl, n-Butyl, iso-Butyl, sec-Butyl und tert-Butyl, insbesondere aus Methyl, Ethyl und n-Butyl.

Ganz besonders bevorzugte Diisocyanate der Formel (A) im Sinne der Erfindung sind die folgenden racemischen Gemische bzw. die folgenden entsprechenden L-Enantiomere:
der Methylester (CAS-Nr. 4460-02-0 als racemisches Gemisch, CAS-Nr. 45158-78-9 in der L-Form), der Ethylester (CAS-Nr. 4254-76-6 als racemisches Gemisch, CAS-Nr. 45172-15-4 als L-Form) und der Butylester (24305-78-0 als racemisches Gemisch, CAS-Nr. 1291098-99-1 als L-Form). Diese ganz besonders bevorzugten Diisocyanate haben eine wirtschaftlich besonders herausragende Bedeutung.

Das erfindungsgemäße Verfahren umfasst wenigstens die Verfahrensschritte 1) und 2). Verfahrensschritt 1 wird weiterhin eine von drei Varianten 1, 2 und 3 vorangestellt, um das Zwischenprodukt der Formel (B) bereitzustellen. Das erfindungsgemäße Verfahren umfasst optional weitere Verfahrensschritte, die während, zwischen und/oder nach den Verfahrensschritten 1) und 2) durchgeführt werden.

In Verfahrensschritt 1) wird das Zwischenprodukt der Formel (B) 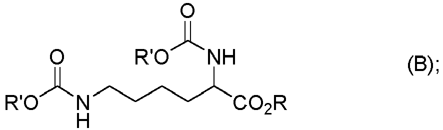 wobei R und jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, bereitgestellt.

R und R` werden bevorzugt aus der Gruppe bestehend C1-C8-Alkyl ausgewählt, mehr bevorzugt aus Methyl, Ethyl, *iso*-Propyl, *n*-Propyl, *n*-Butyl, *iso*-Butyl, s*e*c-Butyl und *tert*-Butyl, insbesondere aus Methyl, Ethyl und n-Butyl.

Das Zwischenprodukt der Formel (B) kann auch in einem geeigneten Reaktionsbehälter vorgelegt werden und so bereitgestellt werden. Im Stand der Technik sind Herstellungsverfahren des Zwischenprodukts der Formel (B) bekannt.

In Verfahrensschritt 2) wird das Zwischenprodukt der Formel (B) thermolytisch gespalten. Dadurch wird das Diisocyanat der Formel (A) erhalten. Die thermolytische Spaltung wird vorzugsweise durch einen Mediator vermittelt, mehr vorzugsweise durch einen metallbasierten Katalysator, noch mehr vorzugsweise durch ein Zinn(II)-Salz. Besonders bevorzugt wird ein Zinn(II)-halogenid, wie Zinn(II)-chlorid oder Zinn(II)-bromid, eingesetzt. Ein Mediator ist im Sinne der Erfindung eine Verbindung, die die thermolytische Spaltung entweder ermöglicht oder idealerweise beschleunigt. Ein Katalysator im Sinne der Erfindung ist eine Verbindung, die die thermolytische Spaltung entweder ermöglicht oder idealerweise beschleunigt und die substöchiometrisch bezogen auf die Edukte (vorliegend das Zwischenprodukt der Formel (B)) eingesetzt werden kann.

Die thermolytische Spaltung wird optional in einem Lösungsmittel durchgeführt. Dieses optionale Lösungsmittel wird aus der Gruppe bestehend aus nicht protischen Lösemitteln ausgewählt. Das optionale Lösungsmittel ist vorzugsweise wasserfrei. Dies bedeutet, dass die Konzentration von Wasser in dem Lösungsmittel bis zu 1 Gew.-% beträgt, vorzugsweise bis zu 0.1 Gew.-%, mehr vorzugsweise bis zu 0.01 Gew.-%. Das optionale Lösungsmittel ist vorzugsweise hochsiedend, d.h. sein Siedepunkt ist vorzugsweise wenigstens 200 °C, mehr vorzugsweise wenigstens 250 °C. Ein bevorzugtes Beispiel ist Marlotherm SH. Gegebenenfalls vorhandene Lösungsmittel und andere flüchtige Bestanteile werden vor Beginn der thermolytischen Spaltung entfernt, z. B. durch Destillation.

Die Temperatur während der thermolytischen Spaltung liegt vorzugsweise im Bereich von 160 bis 240 °C, mehr vorzugsweise im Bereich von 170 - 230 °C bis, insbesondere im Bereich von 180 bis 220 °C. Das gebildete Diisocyanat wird bevorzugt fortlaufend während der Reaktion aus dem Reaktionsbehälter herausdestilliert.

Die Reaktionsdauer der thermolytischen Spaltung hängt von verschiedenen Parametern ab und kann vom Fachmann entsprechend gewählt werden.

In Variante 1 wird das Zwischenprodukt der Formel (B) durch die folgenden Verfahrensschritte erhalten:
a.1) Bereitstellen von Lysin;
a.2) Reaktion des Lysins mit Harnstoff zur Bildung eines Harnstoffaddukts der Formel (C) 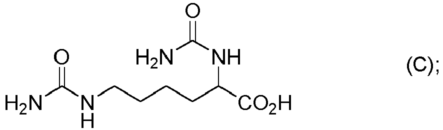 und
a.3) Reaktion des Harnstoffaddukts der Formel (C) mit einem Alkohol zur Bildung des Zwischenprodukts der Formel (B).

In Variante 2 wird das Zwischenprodukt der Formel (B) durch die folgenden Verfahrensschritte erhalten:
b.1) Bereitstellen von Lysin;
b.2) Reaktion des Lysins mit einer Base zur Bildung eines Lysinsalzes der Formel (Z); 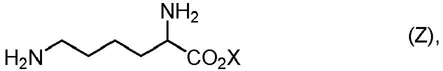 wobei X ein Gegenion darstellt;
b.3) Reaktion des Lysinsalzes der Formel (Z) mit Harnstoff zur Bildung eines Harnstoffsalzes der Formel (Y); 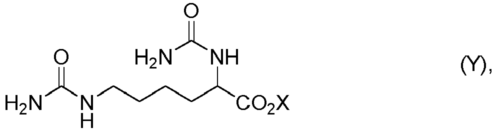 wobei X ein Gegenion darstellt;
b.4) Reaktion des Harnstoffsalzes der Formel (Y) mit einem Alkohol zur Bildung eines Carbamats der Formel (X) 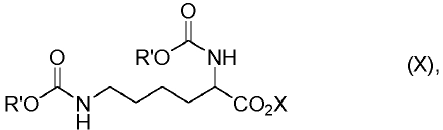
   wobei jedes R` unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, und
   X ein Gegenion darstellt;
b.5) Reaktion des Carbamats der Formel (X) mit einer Säure zur Carbonsäure der Formel (W) 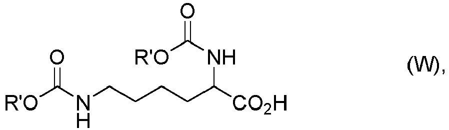 wobei jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, und
b.6) Reaktion der Carbonsäure der Formel (W) mit einem Alkohol zur Bildung des Zwischenprodukts der Formel (B).

In Variante 3 wird das Zwischenprodukt der Formel (B) durch die folgenden Verfahrensschritte erhalten:
c.1) Bereitstellen von Lysin;
c.2) Reaktion des Lysins mit einer Base zur Bildung der eines Lysinsalzes der Formel (Z); 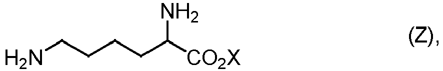 wobei X ein Gegenion darstellt;
c.3) Reaktion des Lysinsalzes der Formel (Z) mit Harnstoff zur Bildung eines Harnstoffsalzes der Formel (Y); 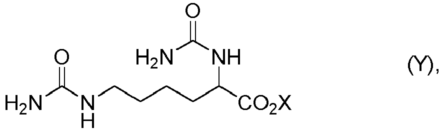 wobei X ein Gegenion darstellt;
c.4) Reaktion des Harnstoffsalzes der Formel (Y) mit einem Alkohol zur Bildung des Zwischenprodukts der Formel (B), ggf. unter vorheriger Protonierung des Carbonsäuresalzes.

Variante 1 umfasst vorteilhafterweise sehr wenige Reaktionsschritte und ist daher und aufgrund der erzielbaren Ausbeuten in den einzelnen Verfahrensschritten wirtschaftlich.

Variante 2 erlaubt die zielgerichtete Einführung einer Vielzahl verschiedener Möglichkeiten für den Rest R. Beispielsweise können so auch höhermolekulare Reste in das Diisocyanat inkorporiert werden, die andernfalls Probleme bei der thermolytischen Spaltung des Zwischenprodukts der Formel (B) verursachen könnten, da sonst in der Regel alle Reste R und R' in dem Zwischenprodukt der Formel (B) identisch sind. Variante 3 erreicht eine geringere Nebenproduktbildung, wenngleich die entstehende Salzfracht abgetrennt werden muss. Bevorzugt werden Variante 1 oder Variante 2 eingesetzt. Mehr bevorzugt wird Variante 1 verwendet.

Die Varianten 1, 2 und 3 dienen dazu, das Zwischenprodukt der Formel (B) bereitzustellen. Diese Varianten ergänzen Verfahrensschritt 1) bzw. werden diesem vorangestellt.

In den Verfahrensschritten a.1), b.1) und c.1) wird Lysin bereitgestellt. Dazu wird es beispielsweise in einem geeigneten Reaktionsbehältnis vorgelegt oder einer entsprechenden Reaktionsmischung beigefügt. Lysin wird hierzu als *L*-Lysin, *R*-Lysin oder als Mischung der vorgenannten (z. B. als Racemat) eingesetzt.

In den Verfahrensschritten a.2), b.3) und c.3) wird Lysin mit Harnstoff zu einem Harnstoffaddukt der Formel (C) bzw. das Lysinsalz der Formel (Z) mit Harnstoff zu einem Harnstoffsalzes der Formel (Y) umgesetzt.

Das Molverhältnis des Harnstoffs (in den Verfahrensschritten a.2), b.3) und c.3) liegt vorzugsweise im Bereich von 1 : 1 bis 5 : 1 bezogen auf die primären Amingruppen des Lysins bzw. des Lysinsalzes der Formel (Z). Dies bedeutet, dass dann rechnerisch auf jede primäre Amingruppe des Lysins bzw. des Lysinsalzes 1 bis 5 Moleküle Harnstoffe eingesetzt werden. So können optimale Ausbeuten der gewünschten Reaktionsprodukte erhalten werden. Mehr vorzugsweise liegt das Molverhältnis im Bereich 1,25 : 1 bis 3 :1, noch mehr vorzugsweise im Bereich von 1,4 bis 2,5.

Vorzugsweise wird die Reaktion in den Verfahrensschritten a.2), b.3) und c.3) in einem polaren Lösungsmittel, insbesondere in Wasser aufgrund seiner Lösungseigenschaften und vorteilhaften ökologischen Charakteristika, durchgeführt.

Die Reaktion in den Verfahrensschritten a.2), b.3) und c.3) wird vorzugsweise bei einer Temperatur von 50 bis 120 °C, mehr vorzugsweise von 80 bis 110 °C, durchgeführt.

Die Reaktion wird typischerweise bis zur vollständigen Umsetzung wenigstens eines der Edukte, meist des Lysins oder des Lysinsalzes der Formel (Z), durchgeführt. Dies kann der Fachmann durch Standardanalytikmethoden prüfen, z. B. gaschromatographisch. Auch das Ende der Ammoniakbildung (nachweisbar beispielsweise durch feuchtes pH-Papier im Abgas) kann als Methode zur Bestimmung der Vollständigkeit der Reaktion dienen. Die Dauer der Reaktion hängt von verschiedenen Parametern, wie beispielsweise der Temperatur, ab. Gängige Reaktionszeiten reichen von 10 min bis 1200 min, vorzugsweise 60 bis 600 min, mehr vorzugsweise 120 bis 420 min.

In Verfahrensschritt a.3), b.6) und c.4) wird aus dem Harnstoffaddukt der Formel (C) bzw. aus der Carbonsäure der Formel (W) bzw. aus dem Harnstoffsalz der Formel (Y) mit einem Alkohol das Zwischenprodukt der Formel (B) gebildet.

Der Alkohol im Sinne der Erfindung ist eine organische Verbindung mit wenigstens einer, vorzugsweise (nur) einer, Hydroxygruppe. Er umfasst entweder eine Alkylgruppe, eine Arylgruppe oder

Molekülfragmenten, die sowohl wenigstens eine Alkylgruppe und wenigstens eine Arylgruppe umfassen, davon, an die die wenigstens eine Hydroxygruppe gebunden ist. Bevorzugt wird der Alkohol aus der Gruppe bestehend C1-C8-Alkohol, mehr bevorzugt aus der Gruppe bestehend aus Methanol, Ethanol, *iso*-Propanol, *n*-Propanol, *n*-Butyl, *iso*-Butanol, *sec*-Butanol und *tert*-Butanol, ausgewählt.

Um eine schnelle und möglichst quantitative Umsetzung des Harnstoffaddukts der Formel (C) bzw. der Carbonsäure der Formel (W) bzw. des Harnstoffsalzes der Formel (Y) zu erreichen, setzt man den einen Alkohol in einen stöchiometrischen Überschuss bezogen auf die genannten Komponenten ein. Mehr bevorzugt liegt das Molverhältnis des einen Alkohols bezogen auf Harnstoffaddukt der Formel (C) bzw. Carbonsäure der Formel (W) bzw. Harnstoffsalz der Formel (Y) im Bereich von 2 : 1 bis 100 : 1. Noch mehr bevorzugt liegt das Molverhältnis im Bereich von 5:1 bis 50 : 1, idealerweise im Bereich von 10 : 1 bis 25 : 1. Wenn mehr als ein Alkohol eingesetzt wird, liegt die Summe aller Stoffmengen der Alkohole in den genannten Bereichen.

Der Druck während der Umsetzung mit dem Alkohol in Verfahrensschritt a.3), b.6), c.4) liegt vorzugsweise mindestens zeitweise im Bereich von ≥ 1 bar, vorzugsweise im Bereich von 1 bis 35 bar, mehr vorzugsweise im Bereich von 1 bis 25 bar.

Vorzugsweise liegt der Druck während der Umsetzung mit dem Alkohol in Verfahrensschritt a.3), b.6), c.4) für 1 bis 20 Stunden, mehr vorzugsweise für 3 bis 10 Stunden, insbesondere für 4 bis 6 Stunden, in dem genannten Bereich.

Die Umsetzung mit dem Alkohol in Verfahrensschritt a.3), b.6), c.4) wird vorzugsweise bei einer Temperatur im Bereich von 150 bis 300 °C, vorzugsweise mit einer Temperatur von 180 bis 230 °C, mehr vorzugsweise mit einer Temperatur von 190 bis 220 °C, durchgeführt. Grundsätzlich gilt für das erfindungsgemäße Verfahren, dass zu hohe Temperaturen in ungünstigen Fällen die Ausbeute durch Decarboxylierung oder ähnliche Zersetzungsreaktionen mindern können. Zu niedrige Temperaturen können unvorteilhafte lange Reaktionszeiten erfordern, um gute Ausbeuten zu erreichen.

Die Reaktion in Verfahrensschritt a.3), b.6), c.4) wird typischerweise bis zum möglichst vollständigen Umsatz des Harnstoffaddukts der Formel (C) bzw. der Carbonsäure der Formel (W) bzw. des Harnstoffsalzes der Formel (Y) geführt. Dazu wird vorzugsweise die Reaktion mit dem Alkohol in Verfahrensschritt a.3), b.6), c.4) für eine Dauer von 1 bis 20 Stunden, vorzugsweise von 3 bis 10 Stunden, insbesondere von 4 bis 6 Stunden in dem genannten Temperaturbereich geführt. Reaktionsdauern außerhalb der genannten können in Abhängigkeit der betreffenden Reaktionskinetik auch vorteilhaft sein.

Optional wird in Verfahrensschritt a.3), b.6), c.4) ein Veresterungskatalysator eingesetzt. Geeignete Veresterungskatalysatoren sind dem Fachmann bekannt. Beispielsweise können Säuren, wie Schwefelsäure, Methansulfonsäure oder Phosphorsäure, sowie metallorganische Verbindungen wie gängige Zinn- oder Titansalze, eingesetzt werden.

In Verfahrensschritt b.2) bzw. c.2) wird Lysin mit einer Base zu einem Lysinsalzes der Formel (Z) umgesetzt. Die Base (in Verfahrensschritt b.2) und c.2)) ist nicht weiter beschränkt. Jede Base, die geeignet ist, das Proton des Lysins zu entfernen und die zur Bildung eines Lysinsalzes der Formel (Z) geeignet ist, kann eingesetzt werden. Dem Fachmann sind hierzu zahlreiche Basen bekannt. Besonders geeignete Basen sind Metallhydroxide (insbesondere Alkalihydroxide wie Lithiumhydroxid, Natriumhydroxid oder Kaliumhydroxid), Amine und Ammoniak.

X (in der Formel (Z)) stellt ein Gegenion dar. X resultiert aus der eingesetzten Base. X ist vorzugsweise aus der Gruppe bestehend aus Alkaliion (insbesondere Ionen von Lithium, Natrium oder Kalium) und Ammoniumion. Wenn X mehrfach positiv geladen ist, bindet es entsprechend an mehrere Anionen des Lysins, z. B. in Mg(Lys)₂.

Die Base in Verfahrensschritt b.2) und c.2) wird typischerweise in einem Molverhältnis von wenigstens 1 : 1 bezogen auf das Lysin eingesetzt. Die erforderliche Menge der Base hängt unter anderem von ihrer Basenstärke ab. Vorzugsweise wird sie in einem Molverhältnis von 1,1 : 1, mehr vorzugsweise in einem Molverhältnis von 1,05 : 1, bezogen auf das Lysin eingesetzt.

Optional wird die Reaktion des Lysins mit einer Base zur Bildung eines Lysinsalzes der Formel (Z) in Verfahrensschritt b.2) und c.2) in einem polaren Lösungsmittel durchgeführt. Geeignete Lösungsmittel werden aus der Gruppe bestehend aus Wasser, Tetrahydrofuran (THF), Dioxan und N,N-Dimethylformamid (DMF) und Mischungen der vorgenannten ausgewählt. Besonders bevorzugt ist Wasser aus den bereits genannten Gründen.

Die Reaktion des Lysins mit einer Base zur Bildung eines Lysinsalzes der Formel (Z) in Verfahrensschritt b.2) oder c.2) wird vorzugsweise bei einer Temperatur im Bereich von 0 bis 50 °C, vorzugsweise mit einer Temperatur von 20 bis 30 °C, mehr vorzugsweise mit einer Temperatur von 20 bis 25 °C, durchgeführt.

Die Reaktion des Lysins mit einer Base zur Bildung eines Lysinsalzes der Formel (Z) (in Verfahrensschritt b.2) und c.2)) wird typischerweise bis zum möglichst vollständigen Umsatz des Lysins geführt. Typische Reaktionsdauern liegen im Bereich von 1 Sekunde bis 3 Stunden, vorzugsweise im Bereich von 0,1 bis 1 Stunde. Die Umsetzung erfolgt in der Regel ohne Zeitverzug (Säure-Base-Reaktion). Lediglich die auftretende Exothermie beschränkt die Reaktionszeit.

In Verfahrensschritt b.4) wird das Harnstoffsalz der Formel (Y) mit einem Alkohol zu einem Carbamat der Formel (X) umgesetzt.

Für die Details und Auswahlmöglichkeiten bezüglich des Alkohols, des Molverhältnisses von Alkohol zu Harnstoffsalz der Formel (Y), der Reaktionsbedingungen (Druck, Temperatur, Reaktionsdauer und optionaler Einsatz eines Veresterungskatalysator) gelten dieselben Angaben wie für die Verfahrensschritte a.3), b.6), c.4).

In einer Ausführungsform der vorliegenden Erfindung werden in Verfahrensschritt b.4) und Verfahrensschritt b.6) unterschiedliche Alkohole verwendet. Vorzugsweise wird dann in Verfahrensschritt b.4) der Alkohol aus der Gruppe bestehend aus Methanol, Ethanol und Butanol und der Alkohol in Verfahrensschritt b.6) aus der Gruppe bestehend aus Methanol, Ethanol und Butanol ausgewählt. Mittels dieser Ausführungsform können auch ansonsten nicht zugängliche Estergruppen in das Diisocyanat eingeführt werden, z. B. Ester ausgehend von Alkoholen, wenn sie an die Carbamatgruppen gebunden sind, die die thermolytische Spaltung des entsprechenden Zwischenprodukts der Formel (B) stören könnten.

In Verfahrensschritt b.5) wird das Carbamat der Formel (X) zur Carbonsäure der Formel (W) umgesetzt. Die Säure (in Verfahrensschritt b.5)) ist nicht weiter beschränkt. Jede Säure, die geeignet ist, aus dem Carbamat der Formel (X) die Carbonsäure der Formel (W) zu bilden, kann erfindungsgemäß eingesetzt werden. Dem Fachmann sind hierzu zahlreiche Säuren bekannt. Vorzugsweise wird die Säure ausgewählt aus Mineralsäuren (vorzugsweise Schwefelsäure, Salzsäure, Phosphorsäure) und organischen Säuren (beispielsweise Methansulfonsäure und Citronensäure). Salzsäure ist besonders bevorzugt.

Die Säure (in Verfahrensschritt b.5)) wird typischerweise in einem Molverhältnis von wenigstens 1 : 1 bezogen auf das Carbamat der Formel (W) eingesetzt. Die erforderliche Menge der Säure hängt unter anderem von ihrer Säurestärke ab. Vorzugsweise wird sie in einem Molverhältnis von 1,2 : 1, mehr vorzugsweise in einem Molverhältnis von 1,05: 1, bezogen auf das Carbamat der Formel (W) eingesetzt.

Optional wird die Reaktion des Carbamats der Formel (X) mit einer Säure zur Carbonsäure der Formel (W) in Verfahrensschritt b.5) in einem polaren Lösungsmittel durchgeführt. Geeignete Lösungsmittel werden aus der Gruppe bestehend aus Wasser, Tetrahydrofuran (THF), Dioxan und N,N-Dimethylformamid (DMF) und Mischungen der vorgenannten ausgewählt. Besonders bevorzugt ist Wasser aus den bereits genannten Gründen.

Die Reaktion des Carbamats der Formel (X) mit einer Säure zur Carbonsäure der Formel (W) in Verfahrensschritt b.5) wird vorzugsweise bei einer Temperatur im Bereich von 0 bis 50 °C, vorzugsweise mit einer Temperatur von 20 bis 30 °C, mehr vorzugsweise mit einer Temperatur von 20 bis 25 °C, durchgeführt. Eine mögliche auftretende Exothermie kann durch Kühlung aufgefangen werden.

Die Reaktion des Carbamats der Formel (X) mit einer Säure zur Carbonsäure der Formel (W) (in Verfahrensschritt b.5)) wird typischerweise bis zum möglichst vollständigen Umsatz des Carbamats der Formel (W) geführt. Typische Reaktionsdauern liegen im Bereich von 1 Sekunde bis 1 Stunde, vorzugsweise im Bereich von 0,1 bis 0,5 Stunde.

In Verfahrensschritt c.4) wird das Harnstoffsalz der Formel (Y) mit einem Alkohol zu dem Zwischenprodukt der Formel (B) umgesetzt. Hierzu wird optional ein Vermittler eingesetzt, der nicht weiter beschränkt ist. Typischerweise weist der Vermittler saure oder wasserziehende Eigenschaften auf. Jeder Vermittler, der geeignet ist, die Reaktion des Harnstoffsalzes der Formel (Y) mit einem Alkohol zur Bildung des Zwischenprodukts der Formel (B) zu vermitteln, kann eingesetzt werden. Dem Fachmann sind hierzu zahlreiche Möglichkeiten bekannt. Beispielsweise können saures Molsieb, starke Mineralsäuren (vorzugsweise Schwefelsäure, Salzsäure, Phosphorsäure) und starke organische Säuren (beispielsweise Methansulfonsäure) eingesetzt werden. Besonders vorteilhaft wird hierzu aus der Gruppe bestehend aus Schwefelsäure, Methansulfonsäure, Salzsäure und Mischungen der vorgenannten ausgewählt.

Der Vermittler (in Verfahrensschritt c.4)) wird typischerweise in katalytischen Mengen bezogen auf das Harnstoffsalz der Formel (Y) eingesetzt. Die erforderliche Menge des Vermittlers hängt unter anderem von seinen Eigenschaften ab. Vorzugsweise wird er in 0,001 - 1 Gew.-% bezogen auf das das Harnstoffsalz der Formel (Y) eingesetzt.

Optional wird die Reaktion des Harnstoffsalzes der Formel (Y) mit einem Alkohol zur Bildung des Zwischenprodukts der Formel (B) (in Verfahrensschritt c.4)) in einem polaren Lösungsmittel durchgeführt. Geeignete Lösungsmittel werden aus der Gruppe bestehend aus Wasser, Tetrahydrofuran (THF), Dioxan und N,N-Dimethylformamid (DMF) und Mischungen der vorgenannten ausgewählt.

Die Reaktion des Harnstoffsalzes der Formel (Y) mit einem Alkohol zur Bildung des Zwischenprodukts der Formel (B) in Verfahrensschritt c.4) wird vorzugsweise bei einer Temperatur im Bereich von 150 - 300 °C, vorzugsweise mit einer Temperatur von 180 bis 230 °C, mehr vorzugsweise mit einer Temperatur von 190 bis 220 °C, durchgeführt.

Die Reaktion des Harnstoffsalzes der Formel (Y) mit einem Alkohol zur Bildung des Zwischenprodukts der Formel (B) in Verfahrensschritt c.4) wird typischerweise bis zum möglichst vollständigen Umsatz des Harnstoffsalzes der Formel (Y) geführt. Typische Dauern der Reaktion liegen im Bereich 1 bis 20 h, vorzugsweise von 3 bis 10h, insbesondere von 4 bis 6h .

Das Verfahren umfasst vorzugsweise einen weiteren Verfahrensschritt (3) nach und/oder während Verfahrensschritt 2):
3) Aufreinigung des Diisocyanats der Formel (A), vorzugsweise durch fraktionierte Destillation.

Besonders bevorzugt wird das Diisocyanat mindestens während der thermolytischen Spaltung durch Destillation, besonders durch fraktionierte Destillation, gereinigt. Dies bedeutet, dass, während sich das Diisocyanat bildet, es bereits aus der Reaktionsmischung entfernt wird. Dadurch wird mitunter die Reaktionsausbeute, insbesondere die Raum-Zeit-Ausbeute, verbessert. Gelegentlich kann es erforderlich sein, dass auch nach Abschluss der thermolytischen Spaltung noch weiter aufgereinigt wird, um eine optimale Ausbeute zu erzielen.

Die Destillation wird bevorzugt unter vermindertem Druck durchgeführt. Bevorzugte Drücke für die Destillation liegen im Bereich von 0,01 bis 200 mbar, bevorzugt im Bereich von 0,1 bis 100 mbar, mehr bevorzugt im Bereich von 1 bis 50 mbar. Dadurch werden möglicherweise thermisch labile Diisocyanate schonender aufgereinigt und so die Ausbeute verbessert. Die Aufreinigung kann alternativ auch nach Abschluss des Verfahrensschritts 2) erfolgen.

In einer bevorzugten Ausführungsform umfasst das erfindungsgemäße Verfahren zur Herstellung eines Diisocyanats der Formel (A) 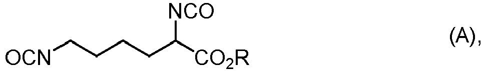 wobei R aus der Gruppe bestehend aus Alkyl, Aryl und Molekülfragmenten, die sowohl wenigstens eine Alkylgruppe und wenigstens eine Arylgruppe umfassen, ausgewählt wird, die folgenden Verfahrensschritte in der angegebenen Reihenfolge:
a.1) Bereitstellen von Lysin;
a.2) Reaktion des Lysins mit Harnstoff zur Bildung eines Harnstoffaddukts der Formel (C) 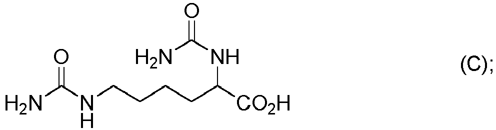 und
a.3) Reaktion des Harnstoffaddukts der Formel (C) mit einem Alkohol zur Bildung des Zwischenprodukts der Formel (B);
   oder
b.1) Bereitstellen von Lysin;
b.2) Reaktion des Lysins mit einer Base zur Bildung eines Lysinsalzes der Formel (Z); 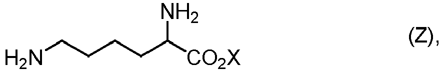 wobei X ein Gegenion darstellt;
b.3) Reaktion des Lysinsalzes der Formel (Z) mit Harnstoff zur Bildung eines Harnstoffsalzes der Formel (Y); 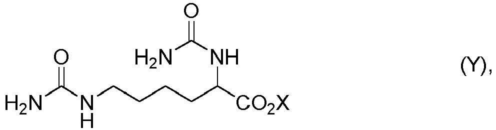 wobei X ein Gegenion darstellt;
b.4) Reaktion des Harnstoffsalzes der Formel (Y) mit einem Alkohol zur Bildung eines Carbamats der Formel (X) 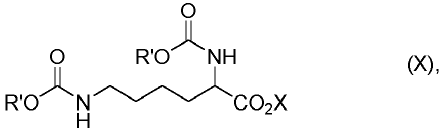
   wobei jedes R` unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, und
   X ein Gegenion darstellt;
b.5) Reaktion des Carbamats der Formel (X) mit einer Säure zur Carbonsäure der Formel (W) 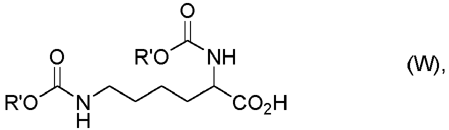 wobei jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, und
b.6) Reaktion der Carbonsäure der Formel (W) mit einem Alkohol zur Bildung des Zwischenprodukts der Formel (B);
   oder
c.1) Bereitstellen von Lysin;
c.2) Reaktion des Lysins mit einer Base zur Bildung eines Lysinsalzes der Formel (Z); 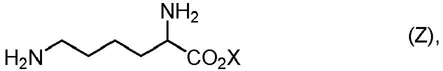 wobei X ein Gegenion darstellt;
c.3) Reaktion des Lysinsalzes der Formel (Z) mit Harnstoff zur Bildung eines Harnstoffsalzes der Formel (Y); 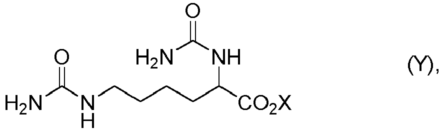 wobei X ein Gegenion darstellt;
c.4) Reaktion des Harnstoffsalzes der Formel (Y) mit einem Alkohol zur Bildung des Zwischenprodukts der Formel (B);
   so dass das Zwischenprodukt der Formel (B) bereitgestellt wird 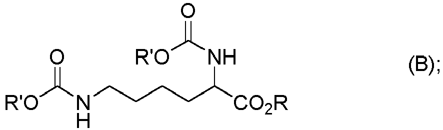 und wobei R und jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird;
2) Thermolytische Spaltung des Zwischenprodukts der Formel (B),
   und optional
3) Aufreinigung des Diisocyanats der Formel (A), vorzugsweise durch fraktionierte Destillation,
   so dass das Diisocyanat der Formel (A) erhalten wird.

Die in dieser Beschreibung und in den Ansprüchen ausgeführten Details und Ausführungsformen bezüglich des erfindungsgemäßen Verfahrens gelten analog für das damit unmittelbar erhaltene Diisocyanat, sofern anwendbar. Zur Vermeidung redundanter Passagen wurden sie hier nicht erneut aufgeführt.

### GEWERBLICHE ANWENDBARKEIT

Das erfindungsgemäße Verfahren und das daraus resultierende Diisocyanat können für eine Vielzahl an Einsatzzwecken verwendet werden. Beispielsweise kann das erfindungsgemäße Verfahren dazu benutzt werden, Diisocyanate bereitzustellen, die in Polyurethanen eingesetzt werden. Solche Polyurethane wiederum können in vielen Industrien eingesetzt werden: Beispiele hierzu sind die Herstellung bioverträglicher Materialien für medizinische Anwendungen oder bioabbaubarer Produkte, die z. B. im Agrarsektor eingesetzt werden, oder allgemein die umweltverträglicher Produkte aus nachwachsenden Rohstoffen.

Die vorliegende Erfindung wird anhand der nachfolgenden Beispiele näher erläutert, ohne den Gegenstand zu beschränken.

### BEISPIELE

Gaschromatographie (GC): Für die GC wurde ein Trace 1300 Gerät mit einer 15 m Zebron ZB-1HT Säule verwendet. Aufgeheizt wurde von 50 auf 270 °C mit 10 °C/min. Die Aminzahl wurde nach DIN 53176:2002 bestimmt. Die Säurezahl wurde nach DIN EN ISO 2114:2002 bestimmt.

### Reaktion 1: Umsetzung von Lysin zum Harnstoffaddukt der Formel (C) (Verfahrensschritt a.2))

584,4 g (4,0 mol) L-Lysin wurden in einem 10L-Druckreaktor vorgelegt (Verfahrensschritt a.1)). Dann wurden 600 g vollentsalztes Wasser, danach 720,7 g (12,0 mol; 1,5 Äquivalente bezogen auf die primären Amingruppen des Lysins) Harnstoff und abschließend 600 g vollentsalztes Wasser zugegeben. Die Reaktionsmischung wurde unter Rühren auf 105 °C aufgeheizt und für 390 min unter Rückfluss erhitzt. Danach wurde die Reaktionsmischung auf Raumtemperatur abgekühlt und das Wasser bei vermindertem Druck abdestilliert. Die so erhaltene Reaktionsmischung wurde in der folgenden Reaktion 2 eingesetzt. Das Produkt ist abgesehen von dem Überschuss Harnstoff laut ¹³C-NMR >90 % rein.

### Reaktion 2: Umsetzung des Harnstoffaddukts der Formel (C) zum Zwischenprodukt der Formel (B) (Verfahrensschritt a.3))

Zu der aus der Reaktion 1 erhaltenen Mischung in dem Druckreaktor wurden 2209,9 g (48,0 mol) Ethanol gegeben. Der Druckreaktor wurde unter Rühren auf 205 °C mittels eines Terematen W-4010 (Lauda LTH 350) aufgeheizt. Dabei stieg der Druck an und wurde bei 25 bar Überdruck für 5 Stunden gehalten, wobei gelegentlich Druck manuell über ein Ventil abgelassen wurde. Nach Ablauf der 5 Stunden wurde die Reaktionsmischung auf Raumtemperatur abgekühlt. Anschließend wurden die flüchtigen Teile unter vermindertem Druck destillativ entfernt. Die Reinheit des Produktes beträgt nach ¹³C-NMR ca. 70 %. Die Aminzahl betrug <1, die Säurezahl 14 mg KOH/g.

### Reaktion 3: Thermolytische Spaltung des Zwischenprodukts der Formel (B) (Verfahrensschritt 2))

250,0 g des aus der Reaktion 2 erhaltenen Produktes (Verfahrensschritt 1)) wurden mit 50 mg von in 50 ml Ethanol gelöstem Zinn(II)chlorid versetzt und in einer Apparatur bestehend aus einem Mehrhalskolben mit aufgesetztem Liebigkühler langsam unter Rühren aufgeheizt, wodurch zunächst Restmengen des Ethanols bei ca. 80 °C Sumpftemperatur abdestilliert wurden. Dann wurde bis zur Schmelze eingeengt. Die Sumpftemperatur betrug hierbei ca. 150 °C. Anschließend wurde der Druck auf 0,5 mbar verringert und die Sumpftemperatur langsam kontinuierlich erhöht. Ab ca. 190 °C Sumpftemperatur bildete sich ein Kondensat des Diisocyanats (Siedepunkt: ca. 110 °C bei dem angegebenen Druck). Die Sumpftemperatur wurde langsam bis 235 °C gesteigert und es wurden insgesamt 60,3 g Diisocyanat aufgefangen (Ausbeute: 34% der Theorie). Das abgetrennte Ethanol landete in der Kühlfalle. Das Produkt war farblos, flüssig und laut GC ca. 95 % rein.

## Patentansprüche

1. Verfahren zur Herstellung eines Diisocyanats der Formel (A) 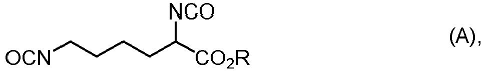 wobei R aus der Gruppe bestehend aus Alkyl, Aryl und Molekülfragmenten umfassend wenigstens eine Alkylgruppe und wenigstens eine Arylgruppe ausgewählt wird, umfassend die Verfahrensschritte in der angegebenen Reihenfolge:
1) Bereitstellen eines Zwischenprodukts der Formel (B) 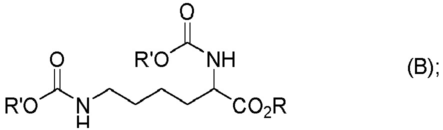 wobei R und jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt werden; und
2) Thermolytische Spaltung des Zwischenprodukts der Formel (B), so dass das Diisocyanat der Formel (A) erhalten wird, **dadurch gekennzeichnet, dass** das Verfahren die folgenden Verfahrensschritte vor Verfahrensschritt 1) umfasst:
a.1) Bereitstellen von Lysin;
a.2) Reaktion des Lysins mit Harnstoff zur Bildung eines Harnstoffaddukts der Formel (C) 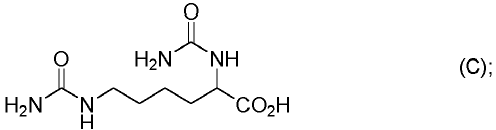 und
a.3) Reaktion des Harnstoffaddukts der Formel (C) mit einem Alkohol zur Bildung des Zwischenprodukts der Formel (B),
oder
b.1) Bereitstellen von Lysin;
b.2) Reaktion des Lysins mit einer Base zur Bildung eines Lysinsalzes der Formel (Z); 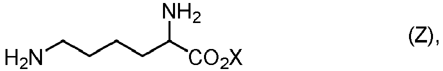 wobei X ein Gegenion darstellt;
b.3) Reaktion des Lysinsalzes der Formel (Z) mit Harnstoff zur Bildung eines Harnstoffsalzes der Formel (Y); 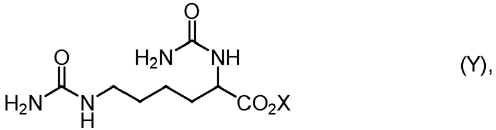 wobei X ein Gegenion darstellt;
b.4) Reaktion des Harnstoffsalzes der Formel (Y) mit einem Alkohol zur Bildung eines Carbamats der Formel (X) 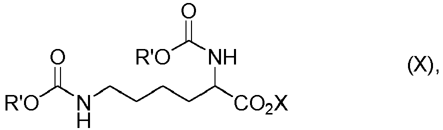
wobei jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird, und
X ein Gegenion darstellt;
b.5) Reaktion des Carbamats der Formel (X) mit einer Säure zur Carbonsäure der Formel (W) 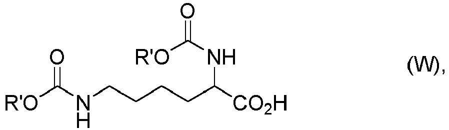 wobei jedes R' unabhängig voneinander aus der Gruppe bestehend aus Alkyl, Aryl und Molekülfragmenten, die sowohl wenigstens eine Alkylgruppe und wenigstens eine Arylgruppe umfassen, ausgewählt wird; und
b.6) Reaktion der Carbonsäure der Formel (W) mit einem Alkohol zur Bildung des Zwischenprodukts der Formel (B),
oder
c.1) Bereitstellen von Lysin;
c.2) Reaktion des Lysins mit einer Base zur Bildung eines Lysinsalzes der Formel (Z); 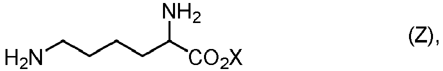 wobei X ein Gegenion darstellt;
c.3) Reaktion des Lysinsalzes der Formel (Z) mit Harnstoff zur Bildung eines Harnstoffsalzes der Formel (Y); 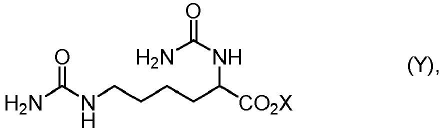 wobei X ein Gegenion darstellt;
c.4) Reaktion des Harnstoffsalzes der Formel (Y) mit einem Alkohol zur Bildung des Zwischenprodukts der Formel (B).

2. Verfahren gemäß einem der vorgenannten Ansprüche, **dadurch gekennzeichnet, dass** R aus der Gruppe bestehend C1-C8-Alkyl, mehr bevorzugt aus Methyl, Ethyl, *iso*-Propyl, *n*-Propyl, *n-*Butyl, *iso*-Butyl, *sec*-Butyl und *tert*-Butyl ausgewählt wird.

3. Verfahren gemäß einem der vorgenannten Ansprüche, **dadurch gekennzeichnet, dass** die thermolytische Spaltung durch einen Mediator vermittelt wird, vorzugsweise durch einen metallbasierten Katalysator, mehr vorzugsweise durch ein Zinn(II)-Salz.

4. Verfahren gemäß einem der vorgenannten Ansprüche, **dadurch gekennzeichnet, dass** das Verfahren einen weiteren Verfahrensschritt 3) nach und/oder während Verfahrensschritt 2) umfasst:
3) Aufreinigung des Diisocyanats der Formel (A), vorzugsweise durch fraktionierte Destillation.

5. Verfahren gemäß einem der vorgenannten Ansprüche, **dadurch gekennzeichnet, dass** das Molverhältnis des Harnstoffs im Bereich von 1 : 1 bis 5 : 1 bezogen auf die primären Amingruppen des Lysins bzw. des Lysinsalzes der Formel (Z) liegt.

6. Verfahren gemäß einem der vorgenannten Ansprüche, **dadurch gekennzeichnet, dass** der Druck während der Umsetzung mit dem Alkohol in Verfahrensschritt a.3), b.4), b.6), c.4) mindestens zeitweise im Bereich von ≥ 1 bar, vorzugsweise im Bereich von 1 bis 35 bar, mehr vorzugsweise im Bereich von 1 bis 25 bar, liegt.

7. Verfahren gemäß Anspruch 6, **dadurch gekennzeichnet, dass** der Druck während der Umsetzung mit dem Alkohol in Verfahrensschritt a.3), b.4), b.6), c.4) für 1 bis 20 Stunden, mehr vorzugsweise für 3 bis 10 Stunden, insbesondere für 4 bis 6 Stunden, in dem genannten Bereich liegt.

8. Verfahren gemäß einem der vorgenannten Ansprüche, **dadurch gekennzeichnet, dass** die Umsetzung mit dem Alkohol in Verfahrensschritt a.3), b.4), b.6), c.4) mindestens bei einer Temperatur von 150 bis 300 °C, vorzugsweise mit einer Temperatur von 180 bis 230°C, mehr vorzugsweise mit einer Temperatur von 190 bis 220 °C, durchgeführt wird.

9. Verfahren gemäß Anspruch 8, **dadurch gekennzeichnet, dass** die Reaktion mit dem Alkohol in Verfahrensschritt a.3), b.4), b.6), c.4) für eine Dauer von 1 bis 20 Stunden, vorzugsweise von 3 bis 10 Stunden, insbesondere von 4 bis 6 Stunden, in dem genannten Temperaturbereich geführt wird.

10. Verfahren gemäß einem der vorgenannten Ansprüche, **dadurch gekennzeichnet, dass** das Molverhältnis des Alkohols bezogen auf Harnstoffaddukt der Formel (C) bzw. Carbonsäure der Formel (W) bzw. Harnstoffsalz der Formel (Y) im Bereich von 2:1 bis 100 : 1 liegt.

11. Verfahren gemäß einem der vorgenannten Ansprüche, **dadurch gekennzeichnet, dass** die Reaktion in den Verfahrensschritten a.2), b.3) und c.3) in einem polaren Lösungsmittel, insbesondere in Wasser, durchgeführt wird.

## Claims

1. Process for preparing a diisocyanate of the formula (A) 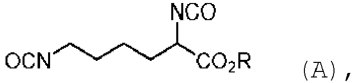 where R is selected from the group consisting of alkyl, aryl and molecular fragments that comprise at least one alkyl group and at least one aryl group, comprising the process steps in the indicated order:
1) providing an intermediate of the formula (B) 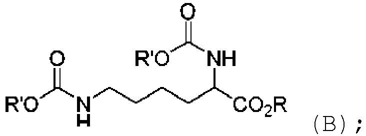 where R and each R' are independently selected from the group consisting of alkyl, aryl and molecular fragments that comprise both at least one alkyl group and at least one aryl group; and
2) thermolytic cleavage of the intermediate of the formula (B),
thereby affording the diisocyanate of the formula (A), **characterized in that** the process includes the following process steps prior to process step 1):
a.1) providing lysine;
a.2) reacting the lysine with urea to form a urea adduct of the formula (C) 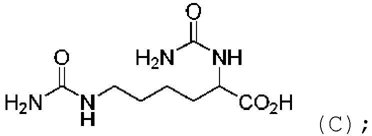 and
a.3) reacting the urea adduct of the formula (C) with an alcohol to form the intermediate of the formula (B),
or
b.1) providing lysine;
b.2) reacting the lysine with a base to form a lysine salt of the formula (Z); 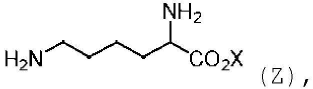 where X is a counterion;
b.3) reacting the lysine salt of the formula (Z) with urea to form a urea salt of the formula (Y); 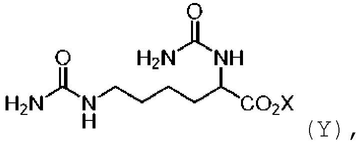 where X is a counterion;
b.4) reacting the urea salt of the formula (Y) with an alcohol to form a carbamate of the formula (X) 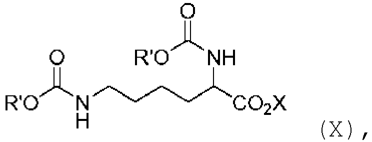
where each R' is independently selected from the group consisting of alkyl, aryl and molecular fragments that comprise both at least one alkyl group and at least one aryl group, and
X is a counterion;
b.5) reacting the carbamate of the formula (X) with an acid to form the carboxylic acid of the formula (W) 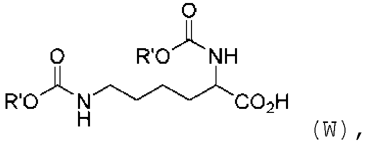 where each R' is independently selected from the group consisting of alkyl, aryl and molecular fragments that comprise both at least one alkyl group and at least one aryl group; and
b.6) reacting the carboxylic acid of the formula (W) with an alcohol to form the intermediate of the formula (B), or
c.1) providing lysine;
c.2) reacting the lysine with a base to form a lysine salt of the formula (Z); 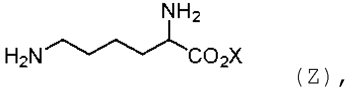 where X is a counterion;
c.3) reacting the lysine salt of the formula (Z) with urea to form a urea salt of the formula (Y); 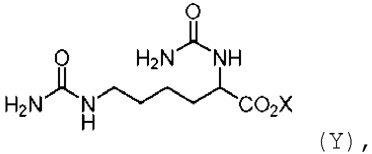 where X is a counterion;
c.4) reacting the urea salt of the formula (Y) with an alcohol to form the intermediate of the formula (B).

2. Process according to any of the preceding claims, **characterized in that** R is selected from the group consisting of C1-C8 alkyl, more preferably from methyl, ethyl, isopropyl, n-propyl, n-butyl, isobutyl, sec-butyl and tert-butyl.

3. Process according to any of the preceding claims, **characterized in that** the thermolytic cleavage is mediated by a mediator, preferably by a metal-based catalyst, more preferably by a tin(II) salt.

4. Process according to any of the preceding claims, **characterized in that** the process includes a further process step 3) after and/or during process step 2):
3) purifying the diisocyanate of the formula (A), preferably by fractional distillation.

5. Process according to any of the preceding claims, **characterized in that** the molar ratio of the urea is within the range from 1:1 to 5:1 relative to the primary amine groups of the lysine or of the lysine salt of the formula (Z).

6. Process according to any of the preceding claims, **characterized in that** the pressure during the reaction with the alcohol in process step a.3), b.4), b.6), c.4) is for at least part of the time within the range from ≥ 1 bar, preferably within the range from 1 to 35 bar, more preferably within the range from 1 to 25 bar.

7. Process according to Claim 6, **characterized in that** the pressure during the reaction with the alcohol in process step a.3), b.4), b.6), c.4) is within the specified range for 1 to 20 hours, more preferably for 3 to 10 hours, in particular for 4 to 6 hours.

8. Process according to any of the preceding claims, **characterized in that** the reaction with the alcohol in process step a.3), b.4), b.6), c.4) is carried out at least at a temperature of from 150 to 300°C, preferably at a temperature of from 180 to 230°C, more preferably at a temperature of from 190 to 220°C.

9. Process according to Claim 8, **characterized in that** the reaction with the alcohol in process step a.3), b.4), b.6), c.4) is executed within the specified temperature range for a period of 1 to 20 hours, preferably of 3 to 10 hours, in particular of 4 to 6 hours.

10. Process according to any of the preceding claims, **characterized in that** the molar ratio of the alcohol relative to the urea adduct of the formula (C) or carboxylic acid of the formula (W) or urea salt of the formula (Y) is within the range from 2:1 to 100:1.

11. Process according to any of the preceding claims, **characterized in that** the reaction in process steps a.2), b.3) and c.3) is carried out in a polar solvent, more particularly in water.

## Revendications

1. Procédé pour la préparation d'un diisocyanate de formule (A) 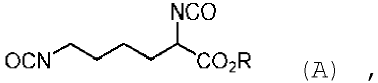 dans laquelle R est choisi dans le groupe constitué par alkyle, aryle et les fragments moléculaires comprenant au moins un groupe alkyle et au moins un groupe aryle, comprenant les étapes de procédé, dans l'ordre indiqué, de :
1) mise à disposition d'un produit intermédiaire de formule (B) 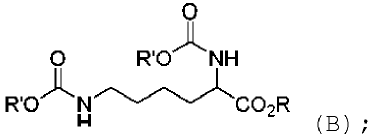 dans laquelle R et chaque R', indépendamment l'un de l'autre, sont choisis dans le groupe constitué par alkyle, aryle et les fragments moléculaires qui comprennent tant au moins un groupe alkyle qu'au moins un groupe aryle ; et
2) dissociation thermolytique du produit intermédiaire de formule (B),
de sorte que le diisocyanate de formule (A) est obtenu, **caractérisé en ce que** le procédé comprend les étapes de procédé suivantes, avant l'étape de procédé 1) :
a.1) mise à disposition de lysine ;
a.2) réaction de la lysine avec de l'urée en vue de la formation d'un produit d'addition d'urée de formule (C) 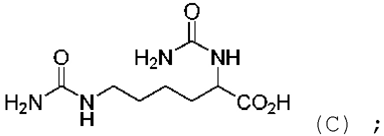 et
a.3) réaction du produit d'addition d'urée de formule (C) avec un alcool en vue de la formation du produit intermédiaire de formule (B),
ou
b.1) mise à disposition de lysine ;
b.2) réaction de la lysine avec une base en vue de la formation d'un sel de lysine de formule (Z) ; 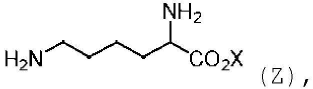 dans laquelle X représente un contre-ion ;
b.3) réaction du sel de lysine de formule (Z) avec de l'urée en vue de la formation d'un sel d'urée de formule (Y) ; 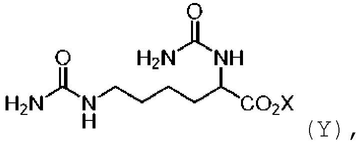 dans laquelle X représente un contre-ion ;
b.4) réaction du sel d'urée de formule (Y) avec un alcool en vue de la formation d'un carbamate de formule (X) 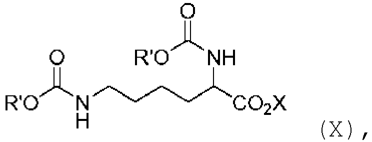
dans laquelle chaque R', indépendamment, est choisi dans le groupe constitué par alkyle, aryle et les fragments moléculaires qui comprennent tant au moins un groupe alkyle qu'au moins un groupe aryle, et
X représente un contre-ion ;
b.5) réaction du carbamate de formule (X) avec un acide en acide carboxylique de formule (W) 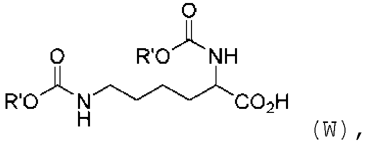 dans laquelle chaque R', indépendamment, est choisi dans le groupe constitué par alkyle, aryle et les fragments moléculaires qui comprennent tant au moins un groupe alkyle qu'au moins un groupe aryle ; et
b.6) réaction de l'acide carboxylique de formule (W) avec un alcool en vue de la formation du produit intermédiaire de formule (B),
ou
c.1) mise à disposition de lysine ;
c.2) réaction de la lysine avec une base en vue de la formation d'un sel de lysine de formule (Z); 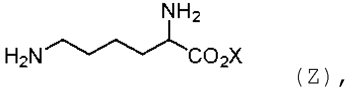 dans laquelle X représente un contre-ion ;
c.3) réaction du sel de lysine de formule (Z) avec de l'urée en vue de la formation d'un sel d'urée de formule (Y) ; 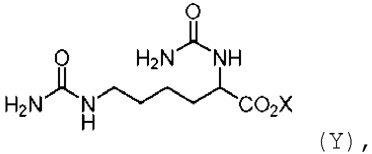 dans laquelle X représente un contre-ion ;
c.4) réaction du sel d'urée de formule (Y) avec un alcool en vue de la formation du produit intermédiaire de formule (B).

2. Procédé selon l'une des revendications susmentionnées, **caractérisé en ce que** R est choisi dans le groupe constitué par C₁-C₈-alkyle, plus préférablement par méthyle, éthyle, *iso*-propyle, *n*-propyle, *n*-butyle, *iso*-butyle, *sec*-butyle et *tert*-butyle.

3. Procédé selon l'une des revendications susmentionnées, **caractérisé en ce que** la dissociation thermolytique est médiée par un médiateur, de préférence par un catalyseur à base de métal, plus préférablement par un sel d'étain (II).

4. Procédé selon l'une des revendications susmentionnées, **caractérisé en ce que** le procédé comprend une autre étape de procédé 3) après et/ou pendant l'étape de procédé 2) :
3) purification du diisocyanate de formule (A), de préférence par distillation fractionnée.

5. Procédé selon l'une des revendications susmentionnées, **caractérisé en ce que** le rapport molaire de l'urée se situe dans la plage de 1 : 1 à 5: 1 par rapport aux groupes amine primaire de la lysine ou, selon le cas, du sel de lysine de formule (Z).

6. Procédé selon l'une des revendications susmentionnées, **caractérisé en ce que** la pression pendant la transformation avec l'alcool dans l'étape de procédé a.3), b.4), b.6), c.4) se situe au moins temporairement dans la plage ≥ 1 bar, de préférence dans la plage de 1 à 35 bars, plus préférablement dans la plage de 1 à 25 bars.

7. Procédé selon la revendication 6, **caractérisé en ce que**, pendant la transformation avec l'alcool dans l'étape de procédé a.3), b.4), b.6), c.4), la pression se situe dans la plage mentionnée pendant 1 à 20 heures, plus préférablement pendant 3 à 10 heures, en particulier pendant 4 à 6 heures.

8. Procédé selon l'une des revendications susmentionnées, **caractérisé en ce que** la transformation avec l'alcool dans l'étape de procédé a.3), b.4), b.6), c.4) est réalisée au moins à une température de 150 à 300°C, de préférence à une température de 180 à 230°C, plus préférablement à une température de 190 à 220°C.

9. Procédé selon la revendication 8, **caractérisé en ce que** la réaction avec l'alcool dans l'étape de procédé a.3), b.4), b.6), c.4) est effectuée dans la plage de température mentionnée pendant une durée de 1 à 20 heures, de préférence de 3 à 10 heures, en particulier de 4 à 6 heures.

10. Procédé selon l'une des revendications susmentionnées, **caractérisé en ce que** le rapport molaire de l'alcool par rapport au produit d'addition d'urée de formule (C) ou, selon le cas, à l'acide carboxylique de formule (W) ou, selon le cas, au sel d'urée de formule (Y) se situe dans la plage de 2:1 à 100 : 1.

11. Procédé selon l'une des revendications susmentionnées, **caractérisé en ce que** la réaction dans les étapes de procédé a.2), b.3) et c.3) est réalisée dans un solvant polaire, en particulier dans de l'eau.
